# EUROPEAN PATENT SPECIFICATION

(11) **EP 1 006 985 B1**
(45) Date of publication and mention of the grant of the patent: **17.04.2002**
(21) Application number: 98903005.1
(22) Date of filing: 14.01.1998
(51) Int. Cl.: A61K 7/00, A61K 7/06, A61K 7/48

(54) **AQUEOUS COMPOSITION COMPRISING BIOTIN-CONTAINING LIPOSOMES**
Wässrige Biotinenthaltende Liposomenzusammensetzung
COMPOSITION AQUEUSE COMPRENANT DES LIPOSOMES CONTENANT DE LA BIOTINE

(30) Priority: 18.01.1997 DE 19701651
(43) Date of publication of application: 14.06.2000
(73) Proprietor: Rovi GmbH Handelsgesellschaft für Rohstoffe, 36381 Schlüchtern (DE)
(72) Inventor: TEICHMÜLLER, Ernst, E., D-36381 Schlüchtern (DE); BLUME, Gabriele, D-36396 Steinau (DE)
(74) Representative: Sternagel, Fleischer, Godemeyer & Partner Patentanwälte
(86) International application number: EP9800175
(87) International publication number: WO9831326

(56) References cited:
- EP-A- 0 370 491
- EP-A- 0 530 862
- DE-A- 19 509 036
- FR-A- 2 714 600
- US-A- 5 523 078
- F. NOSER: "Biotin: ein Wirkstoff zur Verbesserung der Qualität des Haarkeratins." SEIFEN, ÖLE, FETTE UND WACHSE, vol. 8, 1996, pages 511-515, XP002067226 cited in the application

## Description

The present invention relates to an aqueous composition comprising liposomes in which biotin is encapsulated, and the use of the composition for the treatment of cutaneous appendages, particularly to promote hair growth.

Hair loss, whether it be caused by ageing, a certain illness, or chemotherapy, is a widespread problem. The best-known means, the efficacy of which has been described in connection with the promotion of hair growth, are pyrimidine derivatives such as menoxidile and hormones from the oestrogen group. Both substances are to be classified as pharmaceuticals, and are thus only available on prescription.

The hair growth promoting effects of biotin (vitamin H) has been known for a long time. In a study carried out, it was possible to demonstrate that, after a daily oral dose of 2.5 mg of biotin, there was a significant reduction of hair loss and considerable improvement in the quality of the hair (G.L. Floresheim, "Behandlung brüchiger Nägel mit Biotin", Zeitschrift für Hautkrankheiten, 67 (3), 246-255 (1992)). Biochemical examinations carried out in vitro on human epidermis cells have shown that after dosage with high concentrations of biotin (over and above the normal physiological value), the differentiation of the epidermis cells to the keratinocytes was stimulated (A. Fritsche, Inaugural Dissertation, University of Zurich (1990)). Biotin is of vital significance for the proliferation and differentiation of all cells. It is a coenzyme for carboxylase reactions, and obviously also plays a role in a whole series of further metabolic functions, though these have as yet hardly been researched. Biotin is of especially great significance for tissue with high metabolic activity. The positive effects on the metabolic processes of keratinocytes, which induce constant growth of the hair, are only achieved if there is a sufficient level of biotin in the deep hair root (papilla). This level is achieved via the blood circulation, after biotin has been taken orally. However, since orally ingested biotin can only partially be absorbed in the intestine, far higher quantities of biotin must always be ingested than actually reach the area where it has its effect. The high price of biotin makes the poor bio-availability of orally ingested biotin a distinct disadvantage. Equally disadvantageous is the risk of side effects, which are always a possibility with orally administered active substances.

Possibilities for the topical application of biotin with a view to promoting hair growth have also been investigated. As biotin is barely soluble in both water and ethanol (0.02 weight %, and 0.08 weight % respectively), the effect of biotin in normal cosmetic formulations is questionable. So far, it is not known whether sufficient quantities of biotin can be absorbed from a formulation by the scalp. On pages 511 - 515 of the SÖFW Journal 122, edition 8/96, F. Noser and R. Bimczok describe the effects on hair structure of biotin applied epicutaneously to the scalp. Lotions with a biotin content of between 0.003 and 0.01 weight % were used by 60 men with fine, brittle and healthy hair for 6 months. Although a slight strengthening of the hair was observed, it was not possible to ascertain any significant reduction in hair loss.

DE-A-195 09 036 shows compositions with skin-penetrating carrier particles, the construction of which involves at least one amphiphilic physiologically acceptable substance, and which contain either biotin or one of its salts, and are for the treatment of various skin disorders. The carrier particles can be liposomes, and a preferred composition contains 0.25 weight % carrier particles, 0.25 weight % biotin and 1.5 weight % each of the vitamins A and E. There is no description in DE-A-195 09 036 of an application for the liposomes loaded with biotin on cutaneous appendages such as hair and nails.

EP-A-0 665 002 concerns a protective, nourishing and/or firming cosmetic composition for the simultaneous treatment of the skin's surface and deeper layers, made of a first emulsion of lipid vesicles capable of penetrating into the deeper skin layers and a second emulsion of lipid vesicles capable of penetrating the surface layers of the skin. Each emulsion contains at least one active ingredient, selected from a large group of various active substances. The lipid vesicles can be constructed from a number of substances, including phospholipids. From a wide range of possible active substances, vitamin H, D-panthenol, vitamin E and caffeine are mentioned. EP-A-0 665 002 gives no example of a composition containing phospholipid liposomes loaded with biotin.

EP-A-530 862 relates to a cosmetic composition for topical treatment of the skin comprising a mixture of vitamins E, A, B2, B5 and H. Certain vitamins of the composition might be incorporated in liposome type micro-dispersions.

EP-A-370 491 is directed to a liposome comprising as its membrane constituents a certain monophosphate or a certain diphosphate or both. Various cosmetically acceptable active components can be incorporated into said liposomes, for example vitamins, such as vitamin H.

It is the object of the present invention of provide a biotin-containing composition for the external treatment of cutaneous appendages, in which composition the bio-availability of the biotin is improved, compared to already known compositions for the treatment of cutaneous appendages.

The object is met by an aqueous composition comprising liposomes of phospholipids, in which liposome biotin is encapsulated, characterised in that the weight ratio of phospholipids to biotin is in the range of from 50:1 to 1000:1.

Further, the invention concerns the use of an aqueous composition comprising liposomes of phospholipids, in which liposome biotin is encapsulated, for the external cosmetic treatment of cutaneous appendages on humans or animals, as well as a method for preparing one embodiment of the composition according to the present invention.

Preferred embodiments are described in the claims set out below.

In the sense of the present invention, biotin is understood to be D(+)-biotin, also known as vitamin H, and its salts.

Liposomes are spherical forms (vesicles) with a cover of one or several double layers of lipids (bilayers), which form dispersions with aqueous systems. Liposomes can be loaded with various active substances, as in the case at hand. As the vesicles display amphiphilic characteristics, the encapsulated substances can select their own preferred surroundings. The particle size of the liposomes in the present invention is preferably 70 to 300 nm (diameter), more preferably 150 to 200 nm.

The phospholipids used in the present invention are preferably of soy origin, and have a phosphatidyl choline content of at least 73 weight %; a phosphatidyl choline content of 73 - 79 weight % is especially preferred. It is possible, for example, to use phospholipid fractions from: a) 73 - 79 weight % phosphatidyl choline, 0 - 6 weight % lyso-phosphatidyl choline, about 8 weight % phosphatidic acid, about 4 weight % phosphatidyl ethanolamine, and about 9 weight % other lipids; or b) 90 - 96 weight % phosphatidyl choline and 0 - 6 weight % lyso-phosphatidyl choline; or c) at least 95 weight % phosphatidyl choline and a maximum of 2 weight % lyso-phosphatidyl choline. All these percentages are based on the total weight of phospholipids.

The fatty acid residues of soybean phospholipids are mainly derived from unsaturated fatty acids, with linoleic acid accounting for the main part. A typical fatty acid line up is: 61 - 71 % linoleic acid, 3 - 7 % linolenic acid, 6 - 7 % oleic acid, 10 - 15 % palmitic acid, and 1.5 - 4 % stearic acid. All these percentages are based on the total weight of fatty acid residues.

Hydrogenated and partially hydrogenated phospholipids with a phosphatidyl choline content of at least 73 weight %, preferably 75 - 95 weight % can also be used.

The weight ratio of phospholipids to biotin in the composition of the invention preferably ranges from 50:1 to 1000:1, more preferably from 100:1 to 500:1.

A phospholipid content of 5 - 20 weight %, based on the total weight of the composition, is advantageous. A phospholipid content of 8 - 12 weight % is especially preferred.

The proportion of biotin in the composition according to the present invention is preferably 0.001 - 0.5 weight %, more preferably 0.002 - 0.09 weight %, based on the total weight of the composition

The aqueous composition according to the present invention can contain not just biotin, but also further active ingredients to promote hair growth and/or hair regeneration, either alone or in combination. If this is the case, these active ingredients are also encapsulated in the liposomes, and the weight of these additional actives should preferably account to no more than 10 % of the total weight of the composition. Suitable active ingredients are, for example, D-panthenol, vitamin E and caffeine. An especially preferred embodiment of the invention contains a combination of these three active ingredients just mentioned in addition to biotin.

D-panthenol (provitamin B5) is a multi-active substance, which among other things has the property of making the hair up to 10 % thicker and regenerating damaged hair. Preferably, the proportion of D-panthenol is from 0.1 to 10 %, based on the total weight of the composition.

Vitamin E stimulates hair growth by increasing the micro-circulation. Furthermore, it works as an anti-oxidant, and slows down the photooxidative degradation processes of the keratin and hair pigments. Generally, vitamin E is used in the form of vitamin E acetate, and the preferred proportion of vitamin E acetate in the composition according to the invention is from 0.05 to 5 %, based on the total weight of the composition.

Caffeine is another substance that induces hair growth. This non-hormonal substance inhibits 5-α-reductase, an enzyme which is activated by hormonal hair loss. Preferably, the proportion of caffeine in the composition is from 0.05 to 3 %, based on the total weight of the composition.

In addition to the components already mentioned and water, which generally accounts for the greatest proportion of the weight of the formulation, the composition of the invention can contain one or more types of alcohol. Generally, the proportion of the alcohol(s) is from 1 to 20 %, preferably from 3 to 18 %, based on the total weight of the composition. Ethanol is a particularly suitable alcohol, though i-propanol, for instance, may also be used.

As well as, or instead of, alcohol, the composition of the invention can contain a preservative. Suitable preservatives are, for example, formaldehyde, parabens and Euxyl® K 400. If the preservative is present along with alcohol, then the alcohol proportion is usually only from 1 to 5 weight %.

The balance of the composition according to the present invention to one hundred percent is water.

The previously described proportions of the individual components of the composition of the invention refer to a so-called concentrate, which is diluted at a ratio of 1:3 to 1:15 before final use. A dilution ratio of between 1:3 and 1:10 is preferred. In the diluted composition, the following proportions of biotin and any other optional active ingredients are preferred: biotin 0.006 - 0.009 weight %, D-panthenol 0.3 - 1 weight %, vitamin E 0.2 - 0.9 weight %, and caffeine 0.1 - 0.3 weight %, each based on the total weight of the diluted composition.

The compositions of the invention can be produced in accordance with any method for the preparation of liposome suspensions known to the person skilled in the art, for instance those in the book "Liposomes - a practical approach" published by R. C. New (Oxford University Press, 1990). Those compositions of the invention which contain alcohol can for example be produced by dissolving the phospholipids in alcohol, then adding biotin and any-other alcohol-soluble actives and stirring until these have dissolved. The resulting lipid solution is slowly added to water in which any other actives have been dissolved, and the mixture is then stirred. The liposomes which develop spontaneously are then reduced in size by applying energy supply, e.g. by stirring at high speed, highpressure filtration, ultrasound, extrusion or homogenisation, until the desired particle size is arrived at. If oxygen-sensitive phospholipids are being used, the production process can be carried out partially or entirely under protective gas or at reduced pressure.

The liposomes in which the biotin and any other optional actives are encapsulated act as a carrier system for these actives. This means that the actives, having been applied externally to the appropriate area, can be carried directly through the upper layers of the skin to the place where the hair or other keratin formation originates, where it can then develop its effect without any side effects. The liposomes in the present invention act as a carrier system, to ensure that an adequate level of biotin and, if applicable, other actives is present in the epidermis cells. One advantage of the present invention is that, due to the improved transportation to the point of activity, the biotin and any other optional actives can be used in lower concentrations than have so far generally been used in comparable compositions. On the other hand, the solubility of the biotin is increased in the composition of the invention, with the result that in some cases it can be used epicutaneously in higher concentrations than were previously usual.

An aqueous composition containing phospholipid liposomes in which biotin is encapsulated can be utilised for the external cosmetic treatment of cutaneous appendages on human beings and animals. The term cutaneous appendages applies particularly to human hair and nails, but also covers all animal keratin formations such as scales, tortoiseshell, wool, threads, talons, hooves, claws, horns, antlers, and beaks. The present invention concerns particularly the cosmetic treatment to promote hair growth, and strengthen hair growth and/or regeneration. The advantageous effect of the present invention is achieved by applying the aqueous composition comprising biotin-containing liposomes to the scalp.

The invention will now be illustrated, using a number of examples. The following percentages are all based on weight.

| Example | A | B | C |
|---|---|---|---|
| Phospholipids | 10.00 % | 10.00 % | 10.00 % |
| Ethanol | 16.00 % | 16.00 % | 3.33 % |
| Biotin | 0.09 % | 0.02 % | 0.02 % |
| D-panthenol | 10.00 % | 3.00 % | 3.00 % |
| Vitamin E acetate | 2.50 % | 2.50 % | 2.50 % |
| Caffeine | 3.00 % | 0.50 % | 0.50 % |
| Euxyl® K 400 | - | - | 0.30 % |
| Distilled water | ad. 100 % | ad. 100 % | ad. 100 % |
| | | | |
| Lipid/biotin | 111:1 | 500:1 | 500:1 |

The phospholipids used are a phospholipid fraction consisting of:

| | |
|---|---|
| phosphatidyl choline | (73 - 76 %) |
| lyso-phosphatidyl choline | (0 - 6 %) |
| phosphatidic acid | (< 8 %) |
| phosphatidyl ethanolamine | (< 4 %) |
| and other lipids | (approx. 9 %) |
| (percentages refer to dry substance). | |

All three compositions A, B, and C are produced by first dissolving the phospholipids in ethanol, then adding biotin and vitamin E acetate, and stirring until all the substances are dissolved. The other active ingredients, i.e. D-panthenol and caffeine are dissolved in water. The phospholipid solution which has been prepared is added to this aqueous solution drop by drop at reduced pressure, and the mixture is stirred at high speed for approx. 45 minutes. Size evaluation of the liposomes by means of dynamic light scattering resulted in a particle diameter of 150 to 200 nm. In the case of composition C, the preservative Euxyl® K 400 is added after this.
All three compositions are concentrates which are dissolved in water before use, the ratio being 10:1 (A), or 3:1 (B and C).

## Claims

1. An aqueous composition comprising liposomes of phospholipids, in which liposomes biotin is encapsulated,
**characterised in that**
the weight ratio of phospholipids to biotin is in the range of from 50:1 to 1000:1.

2. The aqueous composition of claim 1,
**characterised in that**
the weight ratio of phospholipids to biotin is in the range of from 100:1 to 500:1.

3. The aqueous composition of claims 1 or 2,
**characterised in that**
the proportion of phospholipids is from 5 to 20 %, based on the total weight of the composition.

4. The aqueous composition of claim 3,
**characterised in that**
the proportion of biotin is from 0.001 to 0.5 %, based on the total weight of the composition.

5. The aqueous composition of any of claims 1 to 4,
**characterised in that**
additionally D-panthenol is encapsulated in said liposomes.

6. The aqueous composition of any of claims 1 to 5,
**characterised in that**
additionally vitamin E is encapsulated in said liposomes.

7. The aqueous composition of any of claims 1 to 6,
**characterised in that**
additionally caffeine is encapsulated in said liposomes.

8. The aqueous composition of any of claims 1 to 7,
**characterised in that**
the composition additionally comprises one or more alcohols.

9. The aqueous composition of claim 8,
**characterised in that**
the proportion of alcohol(s) is from 1 to 20 %, based on the total weight of the composition.

10. An aqueous composition obtainable by diluting the composition of any of claims 1 to 9 with water or any aqueous system at a ratio in the range of from 1:3 to 1:15.

11. Use of an aqueous composition comprising liposomes of phospholipids, in which liposomes biotin is encapsulated, for the external cosmetic treatment of cutaneous appendages on humans or animals.

12. The use of claim 11,
**characterised in that**
the cutaneous appendages are human hair.

13. The use of claim 12,
**characterised in that**
the cosmetic treatment serves to promote hair growth, and/or strengthen and/or regenerate hair.

14. Use of an aqueous composition comprising liposomes of phospholipids, in which liposomes biotin is encapsulated, for the manufacture of a medicament for external treatment of cutaneous appendages on humans or animals.

15. The use of any of claims 11 to 14,
**characterised in that**
the aqueous composition is an aqueous composition according to any of claims 1 to 10.

16. A method for preparing an aqueous composition according to claims 8 or 9, comprising
(a) dissolving phospholipids, biotin and any optional alcohol-soluble ingredients in alcohol,
(b) dissolving any optional water-soluble ingredients in water,
(c) slowly adding the alcoholic solution of step (a) to water or the aqueous solution of stage (b),
(d) stirring of the mixture, and
(e) reducing the size of the liposomes that have developed spontaneously, by means of energy supply.

## Patentansprüche

1. Wässrige Zusammensetzung, enthaltend Liposomen aus Phospholipiden, in denen Biotin eingeschlossen ist, **dadurch gekennzeichnet, dass** das Gewichtsverhältnis von Phospholipiden zu Biotin im Bereich von 50:1 bis 1000:1 liegt.

2. Wässrige Zusammensetzung nach Anspruch 1, **dadurch gekennzeichnet, dass** das Gewichtsverhältnis von Phospholipiden zu Biotin im Bereich von 100:1 bis 500:1 liegt.

3. Wässrige Zusammensetzung nach Anspruch 1 oder 2, **dadurch gekennzeichnet, dass** der Anteil der Phospholipide 5 bis 20%, bezogen auf das Gesamtgewicht der Zusammensetzung, beträgt.

4. Wässrige Zusammensetzung nach Anspruch 3. **dadurch gekennzeichnet, dass** der Anteil des Biotins 0,001 bis 0,5%, bezogen auf das Gesamtgewicht der Zusammensetzung, beträgt.

5. Wässrige Zusammensetzung nach einem der Ansprüche 1 bis 4, **dadurch gekennzeichnet, dass** zusätzlich D-Panthenol in den Liposomen eingeschlossen ist.

6. Wässrige Zusammensetzung nach einem der Ansprüche 1 bis 5. **dadurch gekennzeichnet, dass** zusätzlich Vitamin E in den Liposomen eingeschlossen ist.

7. Wässrige Zusammensetzung nach einem der Ansprüche 1 bis 6, **dadurch gekennzeichnet. dass** zusätzlich Coffein in den Liposomen eingeschlossen ist.

8. Wässrige Zusammensetzung nach einem der Ansprüche 1 bis 7, **dadurch gekennzeichnet, dass** zusätzlich ein oder mehrere Alkohole enthalten sind.

9. Wässrige Zusammensetzung nach Anspruch 8, **dadurch gekennzeichnet, dass** der Anteil des oder der Alkohole 1 bis 20%, bezogen auf das Gesamtgewicht der Zusammensetzung, beträgt.

10. Wässrige Zusammensetzung, erhältlich durch Verdünnung der Zusammensetzung nach einem der Ansprüche 1 bis 9 mit Wasser oder mit einem wässrigen System im Verhältnis von 1:3 bis 1:15.

11. Verwendung einer wässrigen Zusammensetzung, enthaltend Liposomen aus Phospholipiden, in denen Biotin eingeschlossen ist, zur äußerlichen kosmetischen Behandlung von Hautanhangsgebilden des Menschen oder Tieres.

12. Verwendung nach Anspruch 11. **dadurch gekennzeichnet, dass** die Hautanhangsgebilde menschliche Haare sind.

13. Verwendung nach Anspruch 12, **dadurch gekennzeichnet, dass** die kosmetische Behandlung der Förderung des Haarwuchses, der Haarkräftigung und/oder der Haarregeneration dient.

14. Verwendung einer wässrigen Zusammensetzung, enthaltend Liposomen aus Phospholipiden, in denen Biotin eingeschlossen ist, zur Herstellung eines Medikaments zur äußerlichen Behandlung von Hautanhangsgebilden des Menschen oder Tieres.

15. Verwendung nach einem der Ansprüche 11 bis 14, **dadurch gekennzeichnet, dass** die wässrige Zusammensetzung eine wässrige Zusammensetzung nach einem der Ansprüche 1 bis 10 ist.

16. Verfahren zur Herstellung einer wässrigen Zusammensetzung nach Anspruch 8 oder 9. umfassend:
(a) Lösen der Phospholipide, des Biotins und ggf. der weiteren alkohollöslichen Bestandteile in Alkohol,
(b) ggf. Lösen der wasserlöslichen Wirkstoffe in Wasser,
(c) langsame Zugabe der alkoholischen Lösung aus Schritt (a) zu Wasser oder zu der wässrigen Lösung aus Schritt (b),
(d) Rühren der Mischung und
(e) Zerkleinern der spontan entstandenen Liposomen durch Energiezufuhr.

## Revendications

1. Composition aqueuse comprenant des liposomes de phospholipides, liposomes dans lesquels la biotine est encapsulée **caractérisée en ce que** le rapport pondéral phospholipides/biotine est compris entre 50/1 et 1000/1.

2. Composition aqueuse selon la revendication 1, **caractérisée en ce que** le rapport pondéral phospholipides/biotine est compris entre 100/1 et 500/1.

3. Composition aqueuse selon les revendications 1 ou 2, **caractérisée en ce que** la proportion de phospholipides est de 5 % à 20 %, par rapport au poids total de la composition.

4. Composition aqueuse selon la revendication 3, **caractérisée en ce que** la proportion de biotine est de 0,001 % à 0,5 %, par rapport au poids total de la composition.

5. Composition aqueuse selon les revendications 1 à 4, **caractérisée en ce que**, en outre, du D-panthénol est encapsulé dans lesdits liposomes.

6. Composition aqueuse selon les revendications 1 à 5, **caractérisée en ce que**, en outre de la vitamine E est encapsulée dans lesdits liposomes

7. Composition aqueuse selon les revendications 1 à 6, **caractérisée en ce que**, en outre, de la caféine est encapsulée dans lesdits liposomes

8. Composition aqueuse selon les revendications 1 à 7, **caractérisée en ce que** la composition comprend en outre un ou plusieurs alcools.

9. Composition aqueuse selon la revendication 8, **caractérisée en ce que** proportion d'alcool(s) est de 1 % à 20 %, par rapport au poids total de la composition.

10. Composition aqueuse susceptible d'être obtenue en diluant la composition selon l'une quelconque des revendications de 1 à 9 dans de l'eau ou tout système aqueux, dans un rapport compris entre 1/3 et 1/15.

11. Utilisation d'une composition aqueuse comprenant des liposomes de phospholipides, liposomes dans lesquels la biotine est encapsulée, pour le traitement cosmétique externe des phanères chez l'homme ou l'animal.

12. Utilisation selon la revendication 11,
**caractérisée en ce que** les phanères sont les cheveux humains.

13. Utilisation selon la revendication 12,
**caractérisée en ce que** le traitement cosmétique sert à favoriser la pousse des cheveux et/ou à renforcer et/ou régénérer les cheveux.

14. Utilisation d'une composition aqueuse comprenant des liposomes de phospholipides, liposomes dans lesquels la biotine est encapsulée, pour la fabrication d'un médicament destiné au traitement cosmétique externe des phanères chez l'homme ou l'animal.

15. Utilisation selon l'une quelconque des revendications 11 à 14, **caractérisée en ce que** la composition aqueuse est une composition aqueuse selon l'une quelconque des revendications 1 à 10.

16. Méthode de préparation d'une composition aqueuse selon les revendications 8 ou 9, comprenant :
(a) dissolution des phospholipides, de biotine, et de tout ingrédient éventuel soluble dans l'alcool
(b) dissolution de tout ingrédient éventuel hydrosoluble dans de l'eau
(c) addition lente de la solution alcoolique obtenue à l'étape (a) dans l'eau ou la solution aqueuse de l'étape (b)
(d) agitation du mélange, et
(e) réduction de la taille des liposomes formés spontanément, par apport d'énergie.
